# EUROPÄISCHE PATENTSCHRIFT

(11) **EP 0 659 098 B1**
(45) Veröffentlichungstag und Bekanntmachung des Hinweises auf die Patenterteilung: **11.12.1996**
(21) Anmeldenummer: 93919301.7
(22) Anmeldetag: 08.09.1993
(51) Int. Cl.: A61N 1/05, A61N 1/372

(54) **IMPLANTAT ZUR ELEKTROSTIMULATION VON GEWEBE**
IMPLANT FOR ELECTROSTIMULATION OF TISSUES
IMPLANT D'ELECTROSTIMULATION DE TISSUS

(30) Priorität: 09.09.1992 DE 4230181
(43) Veröffentlichungstag der Anmeldung: 28.06.1995
(73) Patentinhaber: Kraus, Werner, Dipl.-Ing., D-80333 München (DE)
(72) Erfinder: KRAUS, Werner, D-80333 München (DE); HERIBERT, Stephan, D-80689 München (DE)
(74) Vertreter: von Bezold, Dieter, Dr.
(86) Internationale Anmeldenummer: EP9302431
(87) Internationale Veröffentlichungsnummer: WO9406509

(56) Entgegenhaltungen:
- FR-A- 2 224 125
- FR-A- 2 504 383
- US-A- 3 918 440
- US-A- 5 010 896
- US-A- 5 036 862

## Beschreibung

Die vorliegende Erfindung geht aus von einem Implantat zur Elektrostimulation von Gewebe, mit einem Aufnehmerspulenteil, das mindestens eine elektrische Wicklung ("Aufnehmerspule") mit zwei Anschlüssen enthält, ferner mit einer die Aufnehmerspule umgebenden Umhüllung aus biologisch verträglichem Material, und mit flexiblen Leitungen zum Koppeln der Anschlüsse der Aufnehmerspule mit Gewebeelektroden.

Solche Implantate sind beispielsweise aus US-A- 3,745,995, US-A-3,918,440 und US-A-4,611,597 bekannt. Sie dienen zur Elektrostimulation von Gewebe durch niederfrequente, im allgemeinen sinusförmige Wechselströme, nach einem von Kraus und Lechner entwickelten Verfahren.

Allen diesen bekannten Implanten ist gemeinsam, daß die Anschlüsse der Aufnehmerspule jeweils fest mit einem ersten Ende einer flexiblen Leitung verbunden, z.B. verschweißt sind und daß das andere, zweite Ende der flexiblen Leitungen jeweils fest mit einer Gewebeelektrode oder einer Anschlußvorrichtung für eine solche verbunden ist.

Für verschiedene Situationen im Behandlungsbereich werden aber im allgemeinen auch Leitungen verschiedener Länge benötigt. Die bekannten Implantate können dieser Forderung nur unzureichend Rechnung tragen: Oft sind die Leitungen entweder zu kurz oder zu lang, so daß das Implantat nicht optimal appliziert werden kann, oder es muß eine größere Anzahl von Implanten mit unterschiedlichen Leitungslängen bereit gehalten werden, was unwirtschaftlich ist.

Die vorliegende Erfindung geht also aus von einem Implantat zur Elektrostimulation von Gewebe mit einem Aufnehmerspulenteil, der mindestens eine elektrische Aufnehmerspulenwicklung, deren Enden jeweils mit mindestens einem Anschluß gekoppelt sind, enthält, und mit flexiblen elektrischen Leitungen zum Verbinden der Anschlüsse mit Gewebeelektroden. Die Erfindung vermeidet den oben geschilderten Nachteil der bekannten Implantate dieser Art dadurch, daß die Anschlüsse der Aufnehmerspule jeweils mit mindestens einem am Aufnehmerspulenteil befestigten und von diesem vorspringenden Teil einer Verbindungsvorrichtung verbunden sind und daß die flexiblen Leitungen jeweils ein Ende aufweisen, das zum Verbindungsvorrichtungsteil komplentär ist und elektrisch sowie mechanisch an diesem befestigbar ist.

Ein vorgegebenes Aufnehmerspulenteil kann also bei Bedarf, insbesondere auch noch im OP, mit Leitungen der benötigten Längen und mit den gewünschten Elektroden bzw. Elektrodenanschlußvorrichtungen versehen werden. Die Vorratshaltung von unterschiedlichen Leitungsanordnungen ist wesentlich wirtschaftlicher als die von verschiedenen ganzen Implantaten

Bei einer ersten Ausführungsform der Erfindung können die Anschlußdrähte auf die gewünschte Länge zugeschnitten und dann an entsprechenden Anschlußvorrichtungen der Aufnehmerspule befestigt werden, z.B. durch eine Quetschverbindung oder durch Schrauben ähnlich wie bei einer Lüsterklemme.

Bei einer anderen Ausführungsform der Erfindung ist an jedem Anschluß der Aufnehmerspule mindestens ein kurzes Leiterstück angebracht, das aus der Umhüllung des Implantats herausragt und vorzugsweise aus einem kurzen steifen Stäbchen aus biologisch verträglichem Metall besteht, und an jedem flexiblen Leiterdraht ist eine Hülse angebracht, die durch Quetschen oder eine selbstsperrende Rast- oder Widerhakenanordnung an dem Stäbchen befestigt werden kann.

Die Erfindung wird im folgenden anhand von Ausführungsbespielen unter Bezugnahme auf die Zeichnungen näher erläutert, dabei werden noch weitere Merkmale und Vorteile der Erfindung zur Sprache kommen. Es zeigen:
- Figur 1: eine Draufsicht auf ein gebrauchsfertiges Implantat gemäß einer Ausführungsform der Erfindung;
- Figur 2: eine Draufsicht auf ein Aufnehmerspulenteil eines Implantats der in Figur 1 dargestellten Art;
- Figur 3: eine Schnittansicht des Aufnehmerspulenteils gemäß Figur 2;
- Figur 4: eine Seitenansicht des Aufnehmerspulenteils gemäß Figur 1;
- Figur 5: eine Schnittansicht des Aufnehmerspulenteils gemäß Figur 4;
- Figur 6: eine stark vergrößerte Ansicht einer Anschlußhülse mit einem flexiblen Elektrodenanschlußdraht für ein Aufnehmerspulenteil der in den Figuren 1 bis 5 dargestellten Art;
- Figur 7: eine sogenannte Kielelektrode, die mit einem Anschlußdraht und einer Anschlußhülse der in Figur 6 dargestellten Art versehen ist;
- Figur 8: eine Draufsicht eines weiteren Ausführungsbeispiels eines erfindungsgemäßen Implantats, und
- Figur 9: eine stark vergrößerte Ansicht einer Verbindungsvorrichtung des Implantats gemäß Figur 8.

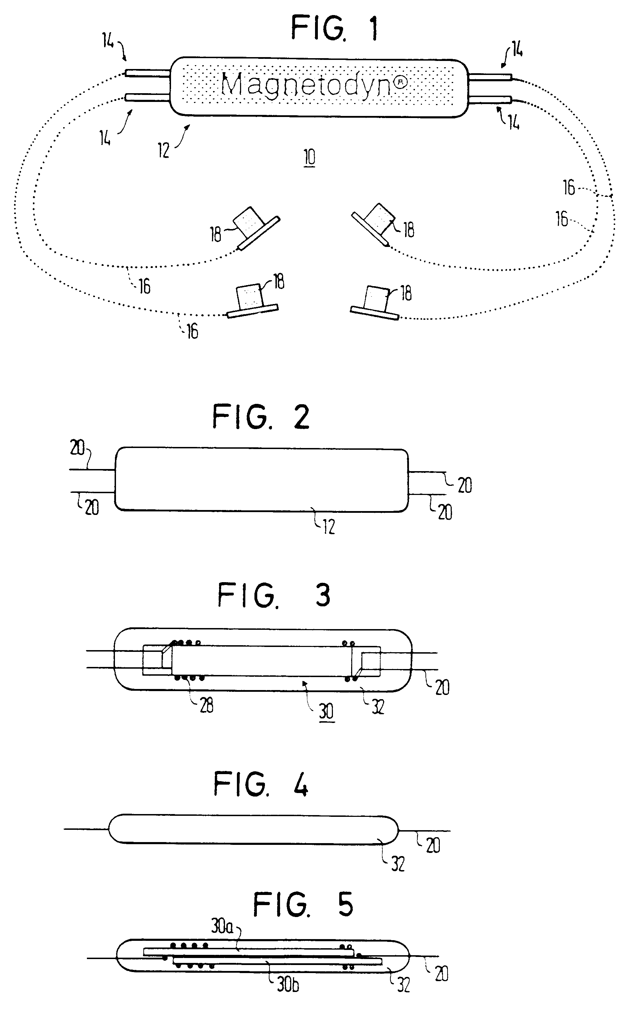

Das in Figur 1 dargestellte Implantat 10 dient zur Elektrostimulation von Gewebe durch einen im wesentlichen sinusförmigen Wechselstrom niedriger Frequenz, insbesondere unter 25 Hz. Es besteht aus einem Aufnehmerspulenteil 12 und Leitungen 16, die mit Elektroden oder Elektrodenanschlußvorrichtungen versehen sind. Das Aufnehmerspulenteil 12 ist mit Anschlußvorrichtungen 14 für mindestens zwei, im Falle der Figur 1 vier flexiblen Leitungen 16 versehen, die in Gewebeelektroden oder Anschlüssen 18 für Gewebeelektroden enden. Die Elektrodenanschlüsse 18 können beispielsweise druckknopfartige Anschlußelemente für Knochenschrauben sein, wie es aus der US 3,918,440 bekannt ist.

Die niederfrequente Wechselspannung wird in einer im Aufnehmerspulenteil enthaltenen Wicklung durch ein magnetisches Wechselfeld induziert, das in bekannter Weise mittels einer äußeren Primärspule erzeugt wird.

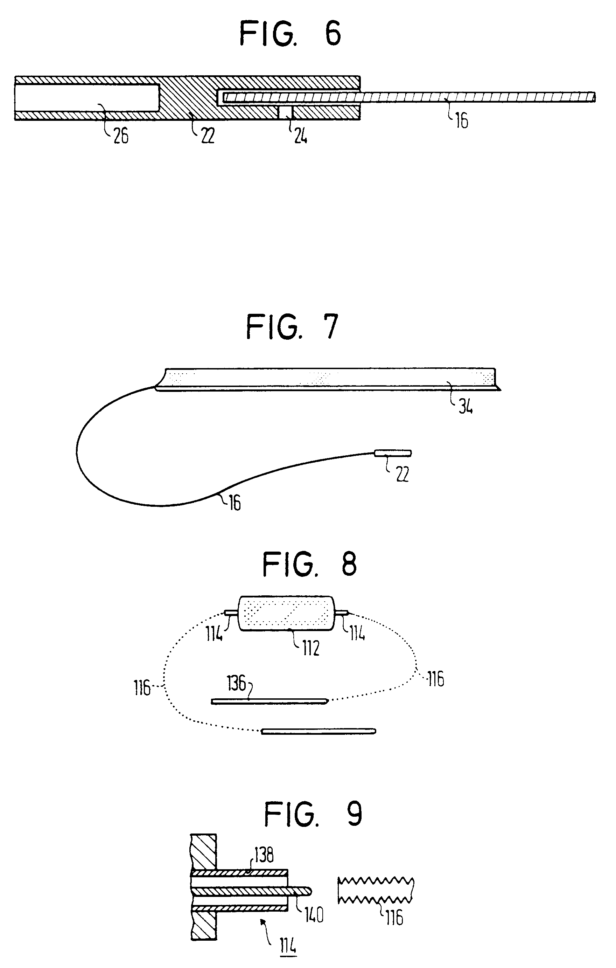

Bei dem Ausführungsbeispiel gemäß Figur 1 bestehen die Verbindungsvorrichtungen aus steifen Drähten 20 (Figur 2) aus gewebeverträglichem Metall, wie einer CrNiMo-Osteosynthesemetallegierung oder einer Titanlegierung, die vom Aufnehmerspulenteil in Längsrichtung vorspringen, und aus Anschlußhülsen an den flexiblen Leitungen. Wie die stark vergrößerte Figur 6 im Schnitt zeigt, ist ein Ende jeder Leitung 16 in einer Hülse 22 aus gewebeverträglichem Metall befestigt, z.B. durch Quetschen an einer hierfür geschwächten Stelle 24. An dem der Leitung 16 abgewandten Ende hat die Hülse 22 ein axiales Loch 26 zur Aufnahme eines entsprechenden Anschlußdrahtes 20 des Aufnehmerspulenteils 12. Die Leitungen 16 bestehen vorzugsweise aus Herzschrittmacher-Leitungsdraht, der sehr flexibel und widerstandsfähig gegen Biegebeanspruchungen ist.

Das Aufnehmerspulenteil 12 enthalt mindestens eine elektrische Wicklung 28 (Figur 3), deren Enden mit jeweils mindestens einem Anschlußdraht elektrisch verbunden sind. Bei der Ausführungsform gemäß Figur 2 bis 5 ist in der Wicklung 28 ein Magnetkern 30 angeordnet, der aus zwei länglichen streifenförmigen Teilen 30a, 30b besteht, die in Längsrichtung etwas gegeneinander versetzt sind, so daß an den Längsenden jeweils ein Stück eines Magnetkernteils übersteht, wie Figur 5 zeigt. Bei der bevorzugten Ausführungsform des vorliegenden Implantats, die in den Figuren 2 bis 5 dargestellt ist, werden die Anschlußdrähte 20 jeweils durch U-förmige Metallbügel gebildet, welche an der Innenseite des an diesem Ende überstehenden Magnetkernteilstückes befestigt, z.B. angeschweißt, und mit einem Ende der Wicklung 28 elektrisch verbunden sind. Wenn die Magnetkernteile 30a, 30b aus elektrisch leitendem Werkstoff, wie Transformatorblech, bestehen, ist zwischen ihnen eine elektrische Isolierung (nicht dargestellt) vorgesehen. Die Wicklung und der Magnetkern sind mit einer Umhüllung 32 aus gewebeverträglichem Material, insbesondere einem gewebeverträglichem Zweikomponenten-Gießharz umgeben.

Außer dem Aufnehmerspulenteil 12 werden dem operierenden Arzt Leitungen 16 unterschiedlicher Längen, die jeweils mit einer Verbindungsvorrichtung, wie einer Hülse 22, und unterschiedlichen Elektroden oder Elektrodenanschlußvorrichtungen versehen sind, zur Verfügung gestellt, so daß der Arzt jeweils die der Operationssituation angemessenen Leitungen am Aufnehmerspulenteil anbringen lassen kann. Hierzu braucht beispielsweise nur die Hülse 22 mit ihrem Loch 26 auf einen Anschlußdraht 20 aufgeschoben und dort fixiert werden, z.B. durch Quetschen oder durch eine zur Fixierung dienende Madenschraube (nicht dargestellt) oder durch eine selbstsperrende Rastanordnung, die beispielsweise durch nach hinten gerichtete sägezahnartige Elemente (nicht dargestellt), die in das Loch 26 vorspringen, gebildet wird.

Figur 7 zeigt ein Beispiel einer Leitung 16, deren eines Ende mit einer Anschlußhülse 22 der in Figur 6 dargestellten Art elektrisch und mechanisch verbunden ist während am anderen Ende elektrisch und mechanisch eine sogenannte Kielelektrode 34 angebracht ist, die aus einem im Querschnitt L- oder T-förmigen Streifen aus gewebeverträglichem Metall besteht (siehe DE 33 40 974 A).

Figur 8 zeigt ein weiteres Ausführungsbeispiel des vorliegenden Implantats, welches sich besonders für Navicular-Anwendungen eignet. Es enthält einen flachen, länglichen Aufnehmerspulenteil 112, an dessen Längsenden sich jeweils eine Verbindungsvorrichtung 114 für eine elektrische Leitung 116 befindet. Bei der Ausführungsform gemäß Fig. 8 ist an jeder Leitung eine Kirschnerdraht-Elektrode 136 angebracht.

Wie Figur 9 zeigt, enthält die Verbindungsvorrichtung eine vorzugsweise aus gewebeverträglichem Material bestehende Hülse 138 und einen metallischen Dorn 140. Der Dorn 140 und vorzugsweise auch die Hülse 138, wenn sie aus Metall besteht, sind mit einem Ende einer nicht dargestellten Wicklung entsprechend der Wicklung 28 (Figur 3) verbunden und so bemessen, daß das Ende einer aus einer Hohllitze bestehenden Anschlußleitung 116 in den Zwischenraum zwischen dem Dorn 140 und der Hülse 138 gesteckt und dort z.B. durch Zusammenquetschen der Hülse 138 fixiert werden kann. Wenn die Hülse aus Metall besteht und mit der Aufnehmerspule verbunden ist, kann der Dorn auch lose sein und in das Ende der Leitung gesteckt werden, bevor es in die Hülse eingeführt wird. Wenn die Leitung nicht hohl, sondern massiv ist oder wenn auch ohne Dorn eine sichere mechanische und elektrische Verbindung der Leitung mit der Hülse 138 gewährleistet ist, kann der Dorn entfallen.

Eventuelle blanke Teile der Hülsen 22, 122 und der Leitungen 16, 116 werden mit einem Isolierschlauch aus gewebeverträglichem Material, inbesondere Silikongummi, überzogen.

## Patentansprüche

1. Implantat zur Elektrostimulation von Gewebe mit einem Aufnehmerspulenteil (12), der mindestens eine elektrische Aufnehmerspulenwicklung (28), deren Enden jeweils mit mindestens einem Anschluß gekoppelt sind, enthält, und mit flexiblen elektrischen Leitungen (16) zum Verbinden der Anschlüsse mit Gewebeelektroden, **dadurch gekennzeichnet**, daß die Anschlüsse der Aufnehmerspule (28) jeweils mit mindestens einem am Aufnehmerspulenteil (12) befestigten und von diesem vorspringenden Teil (20) einer Verbindungsvorrichtung (14) verbunden sind und daß die flexiblen Leitungen (16) jeweils ein Ende aufweisen, das zum Verbindungsvorrichtungsteil komplementär ist und elektrisch sowie mechanisch an diesem befestigbar ist.

2. Implantat nach Anspruch 1, **dadurch gekennzeichnet**, daß das Verbindungsvorrichtungsteii eine Hülse (138) enthält, in der ein Ende einer flexiblen Leitung (116) befestigbar ist.

3. Implantat nach Anspruch 2, **dadurch gekennzeichnet**, daß die elektrische Leitung aus einer hohlen Litze besteht und daß ein Dorn (140) vorgesehen ist, der in das Ende der hohlen Leitung (116) einführbar ist.

4. Implantat nach Anspruch 1, **dadurch gekennzeichnet**, daß der Verbindungsvorrichtungsteil des Aufnehmerspulenteiles (12) aus einem vom Aufnehmerspulenteil vorspringenden steifen Metalldraht (20) besteht und daß an einem Ende jeder flexiblen Leitung (16) eine Hülse (22) angebracht ist, welche an dem Metalldraht befestigbar ist.

5. Implantat nach Anspruch 4, **dadurch gekennzeichnet**, daß die Hülse ein axiales Loch mit einer Widerhakenanordnung enthält, die sich beim Aufschieben der Hülse auf den Metalldraht an diesem festhakt.

6. Implantat nach Anspruch 4, **dadurch gekennzeichnet**, daß die Hülse (22) ein axiales Loch (26) aufweist und durch Quetschen an dem steifen Drahtstück befestigbar ist.

7. Implantat nach einem der vorhergehenden Ansprüche, **dadurch gekennzeichnet**, daß für einen bestimmten Typ von Aufnehmerspulenteil (12, 112) mehrere getrennte flexible Leitungen mit unterschiedlichen Elektroden oder Elektrodenanschlußvorrichtungen vorgesehen sind.

## Claims

1. An implant for electrostimulation of tissues comprising a pick-up coil member (12) which includes at least one electrical pick-up coil winding (28), the ends of which being each coupled to at least one terminal, and flexible electrical leads (16) for coupling said terminals to tissue electrodes, characterized in that the terminals of said pick-up coil (28) are connected to at least one part (20) of a coupling device (14) fixed to and protruding from said pick-up coil member (12) and that each of said flexible leads (16) has an end which is complementary to said coupling device part and adapted to be connected electrically and mechanically to it.

2. An implant according to claim 1, characterized in that said coupling device part comprises a bush (138) within which an end of a flexible lead (116) is fastenable.

3. An implant according to claim 2, characterized in that said electrical lead is comprised by a hollow strand, and that a pin (140) is provided, which is adapted to be inserted into the end of said flexible lead.

4. An implant according to claim 1, characterized in that the coupling device part of said pick-up coil member (12) is comprised by a rigid metal wire (20) protruding from said pick-up coil member, and that a bush (22) which is adapted to be fixed to said metal wire is attached to an end of each flexible lead (16).

5. An implant according to claim 4, characterized in that said bush has an axial hole with barb means, which latches to said metal wire upon pushing said bush onto said wire.

6. An implant according to claim 4, characterized in that said bush (22) has an axial hole (26) and is adapted to be fixed to said rigid wire by crimping.

7. An implant as claimed in any of the preceding claims, characterized in that a plurality of separate flexible leads having different electrodes or electrode coupling means are provided for a predetermined type of pick-up coil member (12, 112).

## Revendications

1. Implant d'electrostimulation de tissus contenant une partie de bobine de recevoir (12) ayant au moins une bobinage électrique de recevoir (28) les extrémités de laquelle sont accouplées à au moins une connexion, et conducteurs électriques flexibles (16) pour joindre les connexions avec électrodes de tissus, characterisée en que les connexions de la bobine de recevoir (28) sont liés avec au moins une partie (20) d'un dispositif de connexion (14) attachée à la partie de bobine de recevoir (12) et saillissent en avant de laquelle, et que les conducteurs flexibles (16) chaque fois auront une extrémité qui est complémentaire à la partie du dispositif de connexion et est électriquement et mécaniquement liable à la même.

2. Implant selont la revendication 1, characterisée en que la partie du dispositif de connexion contient une douille (138) dans laquelle une extrémité d'un conducteur flexible (116) est attachable.

3. Implant selon la revendication 2, characterisée en que le conducteur électrique consiste en un cable torsade creux, et une épine est prévue, qui est à entroduire dans l' extrémité du conducteur creux (116).

4. Implant selon la revendication 1, characterisée en que la partie du dispositif de connection de la partie de bobine de recevoir (12) consiste d'un fil de métal rigide (20) sautant en avant de la partie de bobine de recevoir, et une douille (22), qui est attachable au fil de metal, est installée à une extrémité de chaque conducteur flexible.

5. Implant selon la revendication 4, characterisée en que la douille contient un trou axiale avec un dispositif de crochet, qui s' accroche au fil de metal quand la douille est poussée sur lequel.

6. Implant selon la revendication 4, characterisée en que la douille (22) contient un trou axiale (26) et est apte à attacher à la piece de fil rigide par presser.

7. Implant selon une des revendications antérieures, characterisée en que pour un type défini de partie de bobine de recevoir (12, 112) plusieurs conducteurs flexibles séparés avec des électrodes diiférentes ou des dispositifs à accoupler une électrode différents sont prévues.
